(19) Europäisches Patentamt
European Patent Office
Office européen des brevets

(11) Publication number: 0 560 232 A1

# (12) EUROPEAN PATENT APPLICATION

(21) Application number: **93103605.7**

(22) Date of filing: **05.03.93**

(51) Int. Cl.5: **C07D 311/92**, C07D 405/04, C07D 493/10, A61K 31/35

(30) Priority: **09.03.92 EP 92103980**

(43) Date of publication of application: **15.09.93 Bulletin 93/37**

(84) Designated Contracting States: **AT BE CH DE DK ES FR GB GR IE IT LI LU MC NL PT SE**

(71) Applicant: **HOECHST AKTIENGESELLSCHAFT**

**D-65926 Frankfurt(DE)**

(72) Inventor: **De Souza, Noel, Dr.**
**145, Nibbana, Pali Hill**
**Bandra (W), Bombay 400 050(IN)**
Inventor: **Desai, Premanand, Durgarao, Dr.**
**403, Sea Side Apts., Prabhanagar, P. Balu Marg**
**Prabhadevi, Bombay 400 025(IN)**
Inventor: **Savanur, Shrikant Vishwanath**
**24, Dattatreya, Mith Bunder Road**
**Thane (E) 400 603(IN)**
Inventor: **Chatterjee, Sugata, Dr.**
**H-1/2, Hoechst Ouarters, Darga Road**
**Mulund (W), Bombay 400 082(IN)**
Inventor: **D'Sa, Adolf, Dr.**
**601, Miramar, Veer Savarkar Marg**
**Dadar, Bombay 400 028(IN)**
Inventor: **Joshi, Nitin Sharad, Dr.**
**2/8, Parle Pushpa, Mahant Road (Extn.)**
**Vile Parle (E), Bombay 400 057(IN)**
Inventor: **Vadlamudi, Rao Venkata Satya, Veerabhadra, Dr.**
**Flat 0/6, Chittod Garh, Plot 22, Sector 16 A**
**Vashi, New Bombay 400 705(IN)**
Inventor: **Aroskar, Vijay Atmaram**
**5, Asha-Kiran CHS Ltd, Plot 17/C, Linking Rd Extn.**
**Santacruz (W), Bombay 400 054(IN)**
Inventor: **Rajagopalan, Ramanujam, Dr.**
**B-205, Arati Apts., Sarojini Naidu Road**
**Mulund (W), Bombay 400 080(IN)**
Inventor: **Ghate, Anil Vasantrao**
**2B/7, Garden Apts., Wagle Estate**
**Shivaji Nagar, Thane (W) 400 604(IN)**

(54) **Novel 7-aminolabdanes, a process for their preparation and their use as pharmaceutical agents.**

(57) 7-Aminolabdanes of the formula I

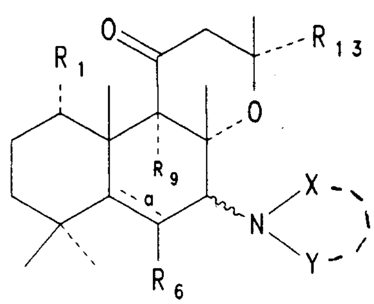

(I)

wherein a, $R_1$, $R_6$, $R_9$, $R_{13}$, X and Y have the given meaning, having blood pressure lowering activity and a process for their preparation are disclosed.

EP 0 560 232 A1

Background of the invention

One of the labdanes of high pharmaceutical interest is the naturally-occurring compound forskolin. Forskolin, coleforsin and semi-synthetic analogues of them are the subject of different patents, patent applications and publications as follows:
US-A-4,088,659;
Indian Patent No. 145 926;
US-A-4,118,508;
US-A-4,152,611;
DE-A-3 535 086;
DE-A-3 623 300;
DE-A-3 718 589;
DE-A-3 735 306;
DE-A-3 740 625;
DE-A-3 740 624;
Tetrahedron Lett. 19 (1977), 1669-1672; J. Chem. Soc., Perkin Trans. 1, (1982) 767;
DE-A-3 346 869;
J. Med. Chem. 31, (1988) 1872.

All the compounds cited in the above-mentioned literature are labdanes wherein the 7-substituent is attached to the 7-position through an oxygen atom. Based on the structural nature of this 7-substituted oxygen function, the compounds are endowed with special attributes that encompass variations in physical properties like water solubility, as well as in biological profile. It is highly desired to obtain new labdanes with improved properties.

This invention pertains to novel 7-aminolabdanes and their derivatives related to forskolin and its analogues, a process for their preparation, and their use as pharmaceutical agents.

Special feature of the invention

It has been now surprisingly found that the naturally-occurring compound forskolin and its analogues can be converted under special reaction conditions, which are part of this invention, to novel 7-amino-7-deacetylforskolin, and to novel 7-amino-7-deacetylforskolin analogues. 7-Amino-7-deacetylforskolin and 7-amino-7-deacetylforskolin analogues can then be converted into new derivatives, all of which form part of this invention. The novel compounds of the invention have unusual biological properties, and are thus useful as novel pharmaceutical and medicinal agents.

Detailed Description of the invention

The present invention describes 7-aminolabdanes of the formula I

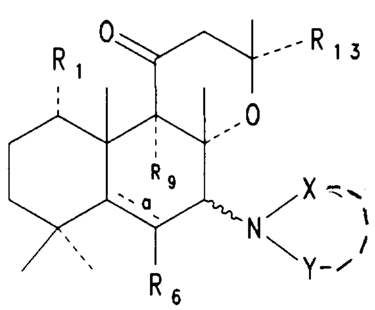

( I )

wherein

| | |
|---|---|
| a | denotes a single bond or a double bond between $C_5$ and $c_6$; |
| $R_1$ = | H or OH; |
| $R_9$ = | H or OH; or |
| $R_1$ and $R_9$ | taken together denote a group selected from |

O S
‖ ‖
-O-C-O, -O-C-O,

$-O-C(C_1-C_4\text{-alkyl})_2-O-$ and

$N(C_1-C_4\text{-alkyl})_2$
|
-O-CH-O-;

$R_6$ =

O O O
‖ ‖ ‖
H, OH, $-OC-(C_1-C_4\text{-alkyl})$, $-OC-(C_1-C_4\text{-alkyl-}NH_2)$, $-O-C-(C_2-C_4\text{-alkenyl})$,

O
‖
$-O-C-(C_2-C_4\text{-alkinyl})$

or $OCONH(CH_2)_m-NH-Q$, wherein

Q = H, $C_1-C_4$-alkyl or aryl;

m denotes an integer from 1 to 20, preferably from 1 - 8, particularly preferred from 1 - 4;

$R_{13}$ = vinyl, ethyl, cyclopropyl or $-CHOHCH_2OH$;

X = H or $(CH_2)_mH$,

Y =

Z
‖
H, $(CH_2)_mH$, CA, $(CH_2)_m$aryl

or aryl, wherein

Z = O, S or NH and

A = H, $(CH_2)_mH$, $(CH_2)_mB$ or NHR' with

B = halogen, OH or

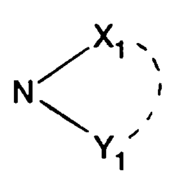 ;

and

R' = H, $C_1$-$C_4$-alkyl or aryl;

wherein

$X_1$ and $Y_1$ each represent an alkyl group or $X_1$ and $Y_1$ together with the nitrogen atom to which they are attached form a heterocycle which may contain an additional heteroatom N, O or S which is optionally substituted by 1, 2 or 3 identical or different alkyl or aryl radicals; or

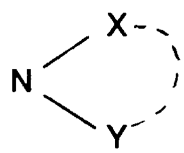

represents a group selected from

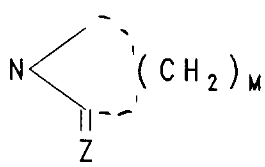 , 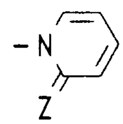 , 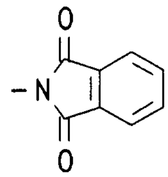

and

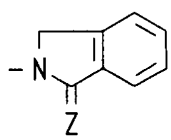

wherein M denotes an integer from 3 to 6 and Z denotes O or S;
and pharmaceutically acceptable salts thereof.

More particularly, the invention describes 7-aminolabdanes of the formula I

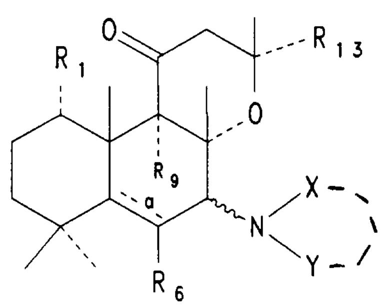

(I)

wherein

$R_1$ = OH;

$R_9$ = OH; or

$R_1$ and $R_9$ taken together denote a group selected from

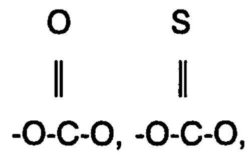

$-O-C(C_1-C_4\text{-alkyl})_2-O-$ and

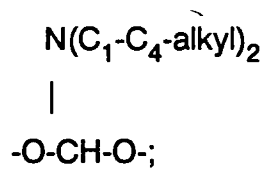

and wherein $R_{13}$, a, $R_6$, X, Y,

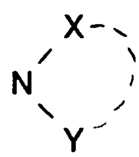

and m, have the same meaning as defined above.

More particularly, the invention also relates to 7-aminolabdanes of the formula I wherein
$R_1$ and $R_9$ denote H and
$R_{13}$, a, $R_6$, X, Y and

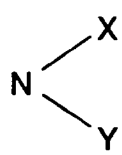

have the previously defined meanings.

In particular preferred are the afore-mentioned compounds of the formula I, wherein $R_{13}$ = vinyl.

Suitable examples of the salts of the compounds according to the invention are the hydrochloride, hydrobromide, sulfate, phosphate, acetate, oxalate, tartrate, citrate, maleate or fumarate.

The term alkyl relates to straight-chain or branched saturated hydrocarbon radicals having 1 to 4 carbon atoms such as for example methyl, ethyl, propyl and t-butyl. $C_2$-$C_4$-alkenyl denotes preferably vinyl and $C_2$-$C_4$-alkinyl denotes preferably propargyl.

An aryl group is preferably a phenyl group which can be substituted by 1, 2 or 3 identical or different substituents such as halogen, $C_1$-$C_3$-alkyl, $C_1$-$C_3$-alkoxy, hydroxy, nitro, amino, trifluoromethyl, cyano and azido.

The term heterocycle represents preferably morpholino, piperidino, pyrrolidino, piperazine and n-methyl piperazino.

The term halogen stands for chlorine, bromine or iodine.

In the formulae depicted here, the various substituents are shown as connected to the labdane nucleus in one to three modes of representation: a full line (—) which indicates a substituent in the $\beta$-orientation (i.e. above the plane of the molecule), and a broken line (----) which indicates a substituent in the $\alpha$-orientation (i.e. below the plane of the molecule) and a wavy line (~~~) which indicates that a substituent may be either $\alpha$- or $\beta$-oriented. All the formulae are drawn in such a way that they depict the compounds in their absolute stereochemical configuration. Where the starting materials having a labdane nucleus are naturally occurring or are derived from natural products they have, as do the final products, a labdane nucleus in the single absolute configuration depicted here. However, the process according to the invention is also meant for the

synthesis of labdanes of the racemic series.

In addition to the optical centers of the labdane nucleus, the substituents thereon may also have chiral centers which contribute to the optical properties of the compounds to the invention and allow their separation by conventional methods, for example by the use of optically active acids. A wavy line (~~) connecting a group to a chiral center indicates that the stereochemistry of the center is unknown, i.e. the group may be present in either of the possible orientations. This invention embraces all the optical isomers and racemic forms of the compounds according to the invention where such compounds have chiral centers in addition to those of the labdane nucleus.

Preferred chemical compounds of the formula I

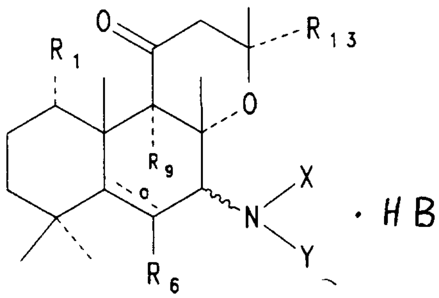

(I)

are shown in the table below as follows:

| $R_1$ | $R_9$ | $R_6$ | $R_{13}$ | a | X | Y | HB | MP [°C] |
|---|---|---|---|---|---|---|---|---|
| OH | OH | H | vinyl | double bond | H | H | - | 234-235 |
| OH | OH | H | vinyl | double bond | H | $COCH_3$ | - | - |
| OH | OH | H | vinyl | double bond | H | $COCH_2Cl$ | - | - |
| OH | OH | H | vinyl | double bond | H | $COCH_2CH_2CH_2Cl$ | - | - |
| OH | OH | H | vinyl | double bond | H | $COCH_2CH_2CH_2OH$ | - | - |
| OH | OH | H | vinyl | double bond | H | 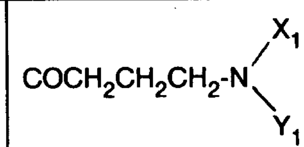 | - | - |
| OH | OH | H | vinyl | double bond | H | $CH_3$ | - | - |
| OH | OH | H | vinyl | double bond | $CH_3$ | $CH_3$ | - | - |
| OH | OH | H | vinyl | double bond | H | $CH_2C_6H_5$ | - | - |
| OH | OH | H | vinyl | double bond | H | $C_6H_5$ | - | - |
| OH | OH | H | vinyl | double bond | H | 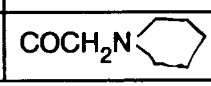 | - | 185-186 |

| $R_1$ | $R_9$ | $R_6$ | $R_{13}$ | a | X | Y | HB | MP [°C] |
|---|---|---|---|---|---|---|---|---|
| OH | OH | H | vinyl | double bond | H | $COCH_2N(CH_3)_2$ | - | 135-136 |
| OH | OH | H | vinyl | double bond | $-\underset{O}{\overset{\parallel}{C}}-CH_2CH_2CH_2$ | | - | >290 |
| OH | OH | H | vinyl | double bond | H | $CONH_2$ | - | - |
| OH | OH | H | vinyl | double bond | H | $C(=NH)-NH_2$ | - | - |
| OH | OH | OH | vinyl | single bond | H | H | - | - |
| OH | OH | OH | vinyl | single bond | H | H | HCl | - |
| OH | OH | OH | vinyl | single bond | H | $COCH_3$ | - | - |
| OH | OH | OH | vinyl | single bond | $-\underset{O}{\overset{\parallel}{C}}-CH_2CH_2CH_2-$ | | - | - |
| OH | OH | $OCOCH_3$ | vinyl | single bond | H | H | - | - |
| OH | OH | $OCOCH_3$ | vinyl | single bond | H | H | HCl | - |
| OH | OH | $OCOCH=CH_2$ | vinyl | single bond | H | H | - | - |
| OH | OH | $OCOCH=CH_2$ | vinyl | single bond | H | H | HCl | - |

| $R_1$ | $R_9$ | $R_6$ | $R_{13}$ | a | X | Y | HB | MP [°C] |
|---|---|---|---|---|---|---|---|---|
| OH | OH | $OCOCH=CH_2$ | vinyl | single bond | H | $COCH_3$ | - | - |
| OH | OH | $OCOCH=CH_2$ | vinyl | single bond | H | $COCH_3$ | HCl | - |
| $-O-C(CH_3)_2-O-$ | | H | vinyl | double bond | H | H | - | Oil |
| " | | H | vinyl | double bond | H | H | HCl | 158-159 |
| " | | H | vinyl | double bond | H | $COCH_3$ | - | - |
| " | | H | vinyl | double bond | H | $COCH_2Cl$ | - | 210 |
| " | | H | vinyl | double bond | H | $COCH_2CH_2CH_2Cl$ | - | 167 |
| " | | H | vinyl | double bond | H | $COCH_2CH_2CH_2OH$ | - | - |
| " | | H | vinyl | double bond | H | $X_1$<br>$COCH_2CH_2CH_2$-N<br>$Y_1$ | - | - |
| " | | H | vinyl | double bond | H | $CH_3$ | - | - |
| " | | H | vinyl | double bond | $CH_3$ | $CH_3$ | - | - |
| " | | H | vinyl | double bond | H | $CH_2C_6H_5$ | - | - |

| $R_1$ | $R_9$ | $R_6$ | $R_{13}$ | a | X | Y | HB | MP [°C] |
|---|---|---|---|---|---|---|---|---|
| $-O-C(CH_3)_2O-$ | | H | vinyl | double bond | H | $C_6H_5$ | - | - |
| " | | H | vinyl | double bond | H | $COCH_2N$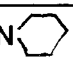 | - | oil |
| " | | H | vinyl | double bond | H | $COCH_2N(CH_3)_2$ | - | oil |
| $-O-C(CH_3)_2-O-$ | | H | vinyl | double bond | $-C(=O)-CH_2CH_2CH_2$ | | – | 212 |
| " | | H | vinyl | double bond | H | $CONH_2$ | - | - |
| " | | H | vinyl | double bond | H | $C(=NH)-NH_2$ | - | - |
| " | | H | vinyl | double bond | 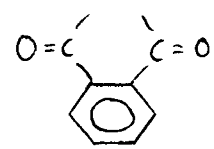 | | - | 92-94 |
| H | H | H | vinyl | double bond | H | H | - | 76-78 |
| H | H | H | vinyl | double bond | H | H | HCl | 169-170 |
| H | H | H | vinyl | double bond | H | $COCH_3$ | - | 168-169 |

| $R_1$ | $R_9$ | $R_6$ | $R_{13}$ | a | X | Y | HB | MP [°C] |
|---|---|---|---|---|---|---|---|---|
| H | H | H | vinyl | double bond | H | $COCH_2Cl$ | - | - |
| H | H | H | vinyl | double bond | H | $COCH_2CH_2CH_2Cl$ | - | 203-205 |
| H | H | H | vinyl | double bond | H | $COCH_2CH_2CH_2OH$ | - | - |
| H | H | H | vinyl | double bond | H | 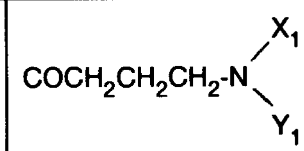 | - | - |
| H | H | H | vinyl | double bond | H | $CH_3$ | - | - |
| H | H | H | vinyl | double bond | $CH_3$ | $CH_3$ | - | - |
| H | H | H | vinyl | double bond | H | $CH_2C_6H_5$ | - | - |
| H | H | H | vinyl | double bond | H | $C_6H_5$ | - | - |
| H | H | H | vinyl | double bond | 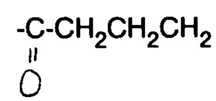 | | - | 162-163 |
| H | H | H | vinyl | double bond | H | $CONH_2$ | - | - |
| H | H | H | vinyl | double bond | H | $C(=NH)-NH_2$ | - | - |

| $R_1$ | $R_9$ | $R_6$ | $R_{13}$ | a | X | Y | HB | MP [°C] |
|---|---|---|---|---|---|---|---|---|
| H | H | H | vinyl | double bond | 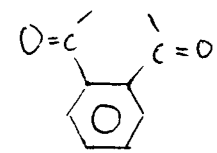 | | - | 161-162 |
| H | H | OH | vinyl | single bond | H | H | - | - |
| H | H | OH | vinyl | single bond | H | H | HCl | - |
| H | H | OH | vinyl | single bond | H | $COCH_3$ | - | - |
| H | H | OH | vinyl | single bond | H | $COCH_2Cl$ | - | - |
| H | H | OH | vinyl | single bond | H | $COCH_2CH_2CH_2Cl$ | - | - |
| H | H | OH | vinyl | single bond | H | $COCH_2CH_2CH_2OH$ | - | - |
| H | H | OH | vinyl | single bond | H | 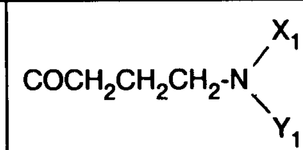 | - | - |
| H | H | OH | vinyl | single bond | H | $CH_3$ | - | - |

| $R_1$ | $R_9$ | $R_6$ | $R_{13}$ | a | X | Y | HB | MP [°C] |
|---|---|---|---|---|---|---|---|---|
| H | H | OH | vinyl | single bond | $CH_3$ | $CH_3$ | - | - |
| H | H | OH | vinyl | single bond | H | $CH_2C_6H_5$ | - | - |
| H | H | OH | vinyl | single bond | H | $C_6H_5$ | - | - |
| H | H | OH | vinyl | single bond | $-\overset{\overset{O}{\|}}{C}-CH_2CH_2CH_2-$ | | - | - |
| H | H | OH | vinyl | single bond | H | $CONH_2$ | - | - |
| H | H | OH | vinyl | single bond | H | $C(=NH)-NH_2$ | | |

The present invention describes a process for the preparation of the novel 7-aminolabdanes of the formula I, which comprises treating a compound of the formula II

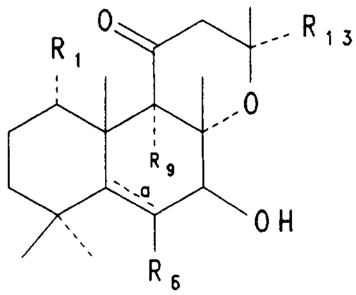

(II)

wherein
$R_6$ and $R_{13}$ have the previously defined meanings, except that $R_6$ = OH,
$R_1$ and $R_9$ = H, or
when $R_9$ = H, $R_1$ = O($C_1$-$C_4$-alkyl),

O
||
OC-($C_1$-$C_4$-alkyl),

O-Si($C_1$-$C_4$-alkyl)$_3$
or $R_1$ and $R_9$ taken together stand for -O-C($C_1$-$C_4$-alkyl)$_2$-O-,

-O-C-O, -O-C-O
||      ||
O      S

or

N ($C_1$-$C_4$-alkyl)$_2$
|
-O-CH-O

in a mixture of a tertiary phosphine such as triphenylphosphine and an imide such as phthalimide or an azide such as hydrazoic acid or zinc azide-pyridine complex in an organic solvent such as tetrahydrofuran, with dialkylazidodicarboxylate such as diethylazidodicarboxylate in an anhydrous aprotic solvent such as ether or tetrahydrofuran. The reaction is generally conducted according to the Mitsunobu reaction [O. Mitsunobu, Synthesis, 1-27(1981), E. Growhowski et al., J. Am. Chem. Soc., 104, (1982) 6876] known to those skilled in the art. The amounts used of the reactants in relation to the compound of the formula II are in excess of molar quantities in the range of 0.1 to 2.0 molar excess. Temperature conditions range from 0-30 °C for a period of 6-30 hours, preferably 18-24 hours. The process further comprises concentrating the reaction mixture, purifying the residue by flash column chromatography over silica gel, using an eluent such as petroleum etherethylacetate (90:10) to obtain:

(a) when an imide is used a 7-imidolabdane of the formula III,

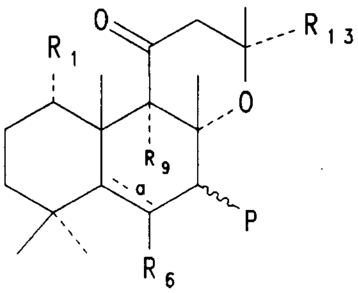

(III)

a) P = imido
b) P = azido

such as the 7-phthalimidolabdane, or
(b) when an azide is used a 7-azidolabdane of the formula III,
wherein $R_1$, $R_9$, $R_6$ and $R_{13}$ have the same meanings as defined in formula II above. The process further comprises treatment of the compound of the formula III wherein P = imido with an appropriate hydrolytic agent, among which are usually used a solution of hydrazine hydrate or methylhydrazine in a solvent such as ethanol, tetrahydrofuran or acetone at a temperature of 0-80°C or in an aqueous solution of methylamine or sodium sulphide for a period of 2-6 hours, or treatment of a compound of the formula III wherein P = azido, first with triphenyl phosphine in solvents such as tetrahydrofuran, benzene, and then with water or with hydrogen in the presence of a catalyst such as platinum oxide, or with metal hydrides such as lithium aluminium hydride or sodium borohydride to yield the 7-aminolabdane of the formula I, wherein $R_7$ = $NH_2$ and $R_1$, $R_9$, $R_6$ and $R_{13}$ have the same meaning as described in the respective formula I.

Compounds of the invention that are derivatives of the 7-$NH_2$ compoundsdescribed above are readily prepared by synthetic reactions known to those skilled in the art. Such skilled persons would recognise that in order to carry out a particular desired synthetic conversion it may be necessary to first protect the reactive sites which may be present in a molecule. Such protection is preferably accomplished by first forming a derivative at the site to be protected, which derivative may be readily re-converted to the original functionality, if desired after the particular synthetic conversion has been carried out. Examples of such protective conversions are esterification or silylation of alcohols, and isoalkylidene/hemiacetal/carbonate formation of diols; other such conversions will suggest themselves to those skilled in the art. Because such techniques are recognised as within the skill of the art, they are not included in the following description of the preparation of the compounds utilized in the method of the invention.

Compounds wherein

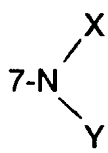

is an acylamino, haloacylamino hydroxyacylamino, aminoacylamino, alkylamino, aralkylamino, arylamino, may be prepared by treating the 7-$NH_2$-labdane with an appropriate acyl-, haloacyl-, hydroxyacyl-, aminoacyl-, alkyl-, aralkyl-, aryl- halide in the presence of an acid appropriate reagent such as pyridine, triethylamine, $K_2CO_3$, NaH or DMAP mixture.

Compounds wherein

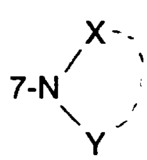

stands for a heterocyclic moiety such as mentioned above may be prepared by treating the appropriate 7-haloacylaminolabdane with a basic agent such as $K_2CO_3$ or NaH.

Compounds wherein

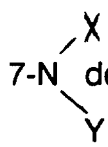 denotes 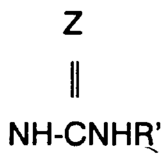

wherein Z and R' has the same meaning as defined above may be prepared by treatment with an appropriate isocyanate or isothiocyanate or $NC\text{-}NH_2$ under the conditions known to those skilled in the art of organic synthesis.

The compound according to the invention and their salts, are useful in the treatment of cardiovascular diseases, metabolic diseases and cancer chemotherapy by virtue of their property to be positive inotropic agents, blood pressure lowering agents, channel modulators, such as potassium channels, chloride channels, calcium channels and the P-glycoprotein multidrug transporter.

The following pharmacological methods were used for the evaluation of the biological activities of the compounds of the invention.

Potassium channel opening activity: - Isolated Rat Portal Vein model.

Normal male albino rats of the Charles Foster strain of weight range 200 to 250 g were used.

After sacrificing the animal, abdomen was opened and portal vein was isolated and mounted in an organ bath containing Kerb's solution. The bath was maintained at 37 °C and aerated with 95 vol.% oxygen and 5 vol.% carbon dioxide gas mixture. The tissue was kept under 0.5 to 1 g tension and allowed to stabilise for about 30 minutes. $KCl_{20}$ and $KCl_{80}$ mM induced cumulative concentrations were measured-through Hugo Sachs Force displacement transducer (K-30) and recorded on 4 channel digital recorder. After taking 2 control responses, the tissue was incubated with test substance for 5 minutes and dose response was repeated. The percent reduction in $KCl_{20}$ and $KCl_{80}$ mM induced contractions were calculated from the control values. Standard potassium channel opener selectively blocks the $KCl_{20}$ induced contractions without affecting the $KCl_{80}$ mM induced responses. If the test compound exhibits selectivity, to confirm further the potassium channel opening activity, glibenclamide a potassium channel blocker induced reversal study was performed. The test compounds were administered in a volume of 0.2 ml per concentration. All results are expressed as mean + SEM.

The results obtained in this model for some of the representative compounds of the invention of the formula I are listed in the table below:

| Compound R1 | R9 | R6 | R13 | a | 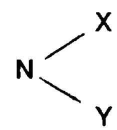 | HB | Vehicle | Dose [Mol] | Percent Inhibition Activity $KCl_{20\ mM}$ | $KCl_{80}$ mM | (n) |
|---|---|---|---|---|---|---|---|---|---|---|---|
| H | H | H | vinyl | double bond | (*a*)$NH_2$ | HCl | D.W. | $10^{-6}$ | 57+9 | 34+9 | 5 |
| H | H | H | vinyl | double bond | (*a*)$NHCOCH_3$- | | DMSO | $10^{-6}$ | 34+10 | 5+2 | 5 |

Result: The split in antispasmodic activity between $K_{20}$ and $K_{80}$ mM of KCl - induced contractions suggests that the compounds exhibit potassium channel opening activity. By virtue of this mode of action, preferred compounds can be potentially used for treating hypertension, asthma, renal colic spasms etc.

Blood Pressure Lowering activity

Hypovolemic rats were used for this activity. Hypovolemic rats were prepared by the administration of 100 mg/kg furosemide intra peritoneally 1 hour prior to the actual experiment. After administering furosemide the rats were anesthesised with 1.5 g/kg urethane given i.p. The right carotid artery was cannulated and blood pressure was mentioned on a physiological recorder by connecting the carotial cannula to a pressure transducer. The left jugular vein was cannulated for i.v. administration of test substances - intra duodenal (i.d.) administration is made by exposing a portion of the duodenum through a small incision made in the abdomen below the sternum. Blood pressure was monitored for 10 minutes in case of i.v. administration and for 90 to 120 minutes when the drug was administered by the i.d. route.

Results obtained for some of the representative compounds of the formula I of the invention are recorded in the table below:

| Compound R1 | R9 | R6 | R13 | a | 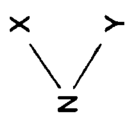 | HB | Dose mg/kg | Route i.v. | Blood pressure (mm Hg) Initial | Post drug | % change | duration (min) |
|---|---|---|---|---|---|---|---|---|---|---|---|---|
| H | H | H | vinyl | double bond | (α)$NH_2$ | HCl | 10 | i.v. | 87±9 | 68±4 | -20.5±3 | 5 |
| H | H | H | vinyl | double bond | (α)$NHCOCH_3$ | - | 30 | i.d. | 94±3 | 80±4 | -15.4±3 | 30 |
| OH | OH | H | vinyl | double bond | (β)$NH_2$ | HCl | 10 | i.v. | 90±10 | 38±2 | 57.8±3 | >70 |

These results indicate a significant fall in the hypovolemic rat blood pressure. This model is indicative of the blood pressure lowering activity of antihypertensive compounds.

The following examples illustrate the preparation of some representative compounds of the invention:

Example 1

8,13-Epoxy-7α-phthalimido-labd-5,14-dien-11-one

8,13-Epoxy-7/β-hydroxy-labd-5,14-dien-11-one (0.636 g, 0.002 M), triphenylphosphine (0.78 g, 0003 M) and phthalimide (0.441 g, 0.003 M) are dissolved in anhydrous tetrahydrofuran (10 ml). To this solution diethylazodicarboxylate (0.47 ml, 0.003 M) in anhydrous tetrahydrofuran (2 ml) is added dropwise with cooling. The resulting solution is stirred at room temperature under $N_2$ atmosphere for 20 hours. Tetrahydrofuran was removed under vacuo and the residue was passed over column of silica gel, using an eluent 5 % ethylacetate - petroleum ether (60-80 °C) to obtain the desired pure compound.
Yield 0.5 g (56%; m.p. = 161-162 °C)
PMR($CDCl_3$):
7.76 (m,4H-aromatic), 5.36 (d of d, $J_{trans}$ = 17 Hz, $J_{cis}$ = 10.8 Hz, 14-vinylic-CH), 5.3 (d, $J_{6,7}$ = 4.8 Hz, 6-CH), 4.68 (d of d, $J_{trans}$ = 17 Hz, $J_{gem}$ = 2 Hz, 15-vinylic-CH),
4.52 (d of d, $J_{cis}$ = 10.8 Hz, $J_{gem}$ = 2 Hz, 15-vinylic-CH), 3.6 (s, 9-CH), 2.4 (d, $J_{6,7}$ = 4.8 Hz, 7-CH), 2.3 (s,12-$CH_2$) 1.56, 1.44, 1.24, 1.16, 1.08 (5x$CH_3$)

Example 2

7α-Amino-8,13-epoxy-labd-5,14-dien-11-one

8,13-Epoxy-7α-phthalimido-labd-5,14-dien-11-one (0.447 g, 0.001M) is dissolved in 5 ml of ethanol and treated with hydrazine hydrate (0.4 ml) under stirring. The reaction mixture is continued to be stirred at 80-85 °C for 4 hours. The precipitated solid is filtered. The filtrate is diluted with water and extracted with ethyl acetate. Organic layer is washed with water, bicarbonate solution, brine, dried over anhydrous sodium sulphate, and evaporated to dryness. The residue obtained was purified over a silica gel column using 10 % acetonitrile-chloroform as an eluent to provide the desired compound. Yield 0.2 g (63 %; m.p. 76-78 °C). The compound is dissolved in dry ether and treated with ethereal hydrogenchloride to obtain the hydrochloride salt, which is crystallised from methylene chloride-petroleum ether yield 0.125 g (m.p. 169-170 °C).
PMR ($CDCl_3$):
5.96 (d of d $J_{trans}$ = 17 Hz, $J_{cis}$ = 10.8 Hz, 14-vinylic-CH), 5.56 (d, $J_{6,7}$ = 4.8Hz, 6-CH), 5.1 (d of d, $J_{trans}$ = 17 Hz, $J_{gem}$ = 2 Hz, 15-vinylic-CH), 4.94 (d of d, $J_{cis}$ = 10.8 Hz, $J_{gem}$ = 2 Hz, 15-vinylic-CH), 3.14 (d, $J_{6,7}$ = 4.8 Hz, 7-CH), 2.72 (s, 9-CH), 2.64 (d, $J_{gem}$ = 2 Hz, 12-$CH_2$), 1.62 (brs, $NH_2$), 1.4, 1.38, 1.32, 1.12, 1.08 (s, 5x$CH_3$)

Example 3

7α-Acetylamino-8,13-epoxy-labd-5,14-dien-11-one

7α-Amino-8,13-epoxy-labd-5,14-dien-11-one (0.3 g) and pyridine (0.5 ml) are dissolved in anhydrous dichloromethane (5 ml). Acetic anhydride (0.5 ml) is then added dropwise and the mixture is stirred for 1 hour. Reaction mixture is washed with water, bicarbonate solution and brine. Dichloromethane layer is separated dried over anhydrous sodium sulphate and evaporated to dryness. The residue obtained is crystallised from dichloromethane - petroleum ether (60-80 °C) to obtain the desired compound. Yield 0.250 g (73 %; m.p. 168-169 °C).
PMR ($CDCl_3$):
5.90 (d of d, $J_{trans}$ = 17 Hz, $J_{cis}$ = 10.8 Hz, 14-vinylic-CH), 5.58 (d, $J_{6,7}$ = 4.8 Hz, 6-CH), 5.14 (d of d, $J_{trans}$ = 17 Hz, $J_{gem}$ = 2 Hz, 15-vinylic-CH), 4.98 (d of d, $J_{cis}$ = 10.8 Hz, $J_{gem}$ = 2 Hz, 15-vinylic-CH), 3.32 (d of d, $J_{6,7}$ = 4.8 Hz, $J_{7,NH}$ = 6.4 Hz, 7-CH), 2.66 (s, 12-$CH_2$), 2.44 (s, 9-CH), 2.06 (s, $OCOCH_3$), 1.52, 1.38, 1.34, 1.16, 1.10 (s, 5x$CH_3$).

Example 4

1α,9α-Isopropylidenyl(1,3-)dioxy-8,13-epoxy-7β-phthalimido-labd-5,14-dien-11-one

Using 1α,9α-isopropylidenyl(1,3-)dioxy-8,13-epoxy-7β-hydroxy-labd-5,14-dien-11-one as starting material, the compound was subjected to the same reaction conditions as described in example 1 to give the

desired compound. Yield 37 %; m.p. 92-94 °C.
PMR ($CDCl_3$):
7.8 (m, 4H-aromatic), 5.68 (d of d, $J_{trans}$ = 17 Hz, $J_{cis}$ = 10.8 Hz, 14-vinylic-CH), 5.64 (d, $J_{6,7}$ = 2.2 Hz, 6-CH), 5.56 (d of d, $J_{trans}$ = 17 Hz, $J_{gem}$ = 2 Hz, 15-vinylic-CH), 5.48 (d, $J_{6,7}$ = 2.2 Hz, 7-CH), 5.0 (d of d $J_{cis}$ = 10.8 Hz, $J_{gem}$ = 2 Hz, 15-vinylic-CH), 4.38 (brt, 1-CH), 3.26 (d, $J_{gem}$ = 18Hz, 12-α-CH), 2.4 (d, $J_{gem}$ = 18Hz, 12-β-CH), 1.54, 1.48, 1.4, 1.36, 1.32, 1.26, 1.16 (s, 7xCH3)

Example 5

7β-Amino-1,9-isopropylidenyl(1,3-)dioxy-8,13-epoxy-labd-5,14-dien-11-one

Using 1α,9α-isopropylidenyl(1,3-)dioxy-8,13-epoxy-7β-phthalimido-labd-5,14-dien-11-one as starting material, the compound was subjected to the same reaction conditions as described in example 2 to give the desired compound as thick viscous oil
(0.750 g, yield 40 %). The compound is dissolved in ether and treated with ethereal hydrogen chloride to obtain hydrochloride salt, which is crystallised from methylene chloride-petroleum ether (60-80 °C); m.p. 158-159 °C.
PMR ($CDCl_3$):
5.98 (d of d, $J_{trans}$ = 17 Hz, $J_{cis}$ = 10.8 Hz, 14-vinylic-CH), 5.42 (d, $J_{6,7}$ = 2.2 Hz, 6-CH), 5.22 (d of d, $J_{trans}$ = 17 Hz, $J_{gem}$ = 2 Hz, 15-vinylic-CH), 4.98 (d of d, $J_{cis}$ = 10.8 Hz, $J_{gem}$ = 2 Hz, 14-vinylic-CH), 4.3 (brt, 1-CH), 4.0 (d, $J_{6,7}$ = 2 Hz, 7-CH), 3.2 (d, $J_{gem}$ = 18 Hz, 12-α-CH), 2.38 (d, $J_{gem}$ = 18 Hz, 12-β-CH), 1.6 (brs, $NH_2$), 1.4, 1.36, 1.24, 1.18, 1.1 (s, 7x$CH_3$)
Two methyls at 1.4 and 1.36.

Example 6

7β-Amino-1α,9α-dihydroxy-8,13-epoxy-labd-5,14-dien-11-one

7β-Amino-1α,9α-isopropylidenyloxy-8,13-epoxy-labd-5,14-dien-11-one hydrochloride (0.25 g, 0.0006 M) is stirred with 2N hydrochloric acid (5 ml) for 2 hours. The reaction mixture is basified to the pH 9 and extracted with ethylacetate. Ethylacetate layer is washed with water, brine, dried over anhydrous sodium sulphate and evaporated to dryness. The residue obtained is crystallised from chloroform and petroleum-ether (60-80 °C); yield 0.120 g (59 %; m.p. 234-235 °C).
PMR($CDCl_3$):
5.96 (d of d, $J_{trans}$ = 17 Hz, $J_{cis}$ = 10.8 Hz, 14-vinylic-CH), 5.42 (d, $J_{6,7}$ = 2.2 Hz, 6-CH), 5.25 (d of d, $J_{trans}$ = 17 Hz, $J_{gem}$ = 2 Hz, 15-vinylic-CH), 4.98 (d of d, $J_{cis}$ = 10.8 Hz, $J_{gem}$ = 2 Hz, 15-vinylic-CH), 4.74 (brt, 1-CH), 4.05 (d, $J_{6,7}$ = 2.2 Hz, 7-CH), 3.05 (d, $J_{gem}$ = 18 Hz, 12-α-CH), 2.64 (d, $J_{gem}$ = 18 Hz, 12-β-CH), 1.52, 1.4, 1.36, 1.30, 1.26 (s, 5x$CH_3$).
Jeol FX 90Q FT NMR Spectrometer used.

Example 7

7α-(N-γ-Chlorobutyryl)amino-8,13-epoxy-labd-5,14-dien-11-one

4-Chlorobutyrylchloride (0,58 ml) is added to a solution of 7-amino-8,13-epoxy-labd-5,14-dien-11-one-(1.0 g) and dry pyridine (0.84 ml) in dry dichlormethane (20 ml). The reaction mixture is stirred overnight at room temperature. The reaction mixture is washed with cold dilute hydrochloric acid, water, sodium bicarbonate solution, water and brine. The dichloromethane layer is separated, dried over anhydrous sodium sulphate and evaporated to dryness. The residue obtained is crystallised from dichloromethane and petroleum ether (60-80 °C) to obtain the desired compound.
Yield 0.850 g (64 %); m.p. 203-205 °C.
PMR ($CDCl_3$):
5.90 (d of d, $J_{trans}$ = 17 Hz, $J_{cis}$ = 10.8 Hz, 14-vinylic-CH), 5,58 (brd, $J_{6,7}$ = 4,8 Hz, 6-CH, NH), 5.12 (d of d, $J_{trans}$ = 17 Hz, $J_{gem}$ = 2 Hz, 15-vinylic-CH), 4.96 (d of d, $J_{cis}$ = 10.8 Hz, $J_{gem}$ = 2 Hz, 15 vinylic-CH),4.32 (d of d, 4.8 Hz, $J_{7CH-NH}$ = 6.4 Hz, 7-CH), 3.64 (t, $J_{CH-CH}$ = 6.3 Hz $CH_2$-Cl), 2.64 (s 12-$CH_2$), 2.44 (s, 9-CH), 2.4-2.3 (m $CH_2$-$CH_2$-Cl), 2.16 (t, $J_{CH-CH}$ = 6.3 Hz, $CH_2$-CO), 1.54, 1.36, 1.34. 1.16, 1.08 (s, 5X$CH_3$).

Example 8

7α-(2-Oxopyrrolidin-1-yl)-8,13-epoxy-labd-5,14-dien-11-one

Sodium hydride (0.15 g , 60 % dispersion) is added to a solution of 7α-(N-γ-chlorobutyryl)amino-8,13-epoxy-labd-5,14-dien-11-one (0.63g) in dry dichloromethane (5 ml) and dry dimethylformamide (1 ml). The reaction mixture is stirred at room temperature for 2 hours. The reaction mixture is washed with water, brine. The dichloromethane layer is separated, dried over sodium sulphate and evaporated to dryness. The residue obtained is crystallised from dichloromethane and petroleum ether (60-80 °C) to obtain the desired compound.
Yield 0.4 g (69 %); m.p. 162 - 163 °C.
PMR ($CDCl_3$):
5.80 (d of d, $J_{trans}$ = 17 Hz, $J_{cis}$ = 10.8 Hz, 14-vinylic-CH), 5.20 (d, $J_{6,7}$ = 4.8 Hz, 6-CH), 5.08 (d of d, $J_{trans}$ = 17 Hz, $J_{gem}$ = 2 Hz, 15-vinylic-CH), 4.92 (d of d, $J_{cis}$ = 10.8 Hz, $J_{gem}$ = 2 Hz, 15-vinylic-CH), 4.80 (d, $J_{6,7}$ = 4.8 Hz, 7-CH), 3.4-3.2 (m, N-$CH_2$-$CH_2$), 2.64 s, 9-CH), 2.58 („12-$CH_2$), 2.4-1.8 (m, CO-$CH_2$-$CH_2$), 1.54, 1.40, 1.36, 1.16, 1.14 (s, 5X $CH_3$).

Example 9

7β-(N-γ-Chlorobutyryl)amino-1,9-isopropylidenyl(1,3-)dioxy-8,13-epoxy-labd-5,14-dien-11-one

The title compound is prepared by following the procedure of Example 7 using 7-β-amino-1,9-isopropylidenyl(1,3-)dioxy-8,13-epoxy-labd-5,14-dien-11-one (0.5g), 4-chlorobutyryl chloride (0.15 ml) and dry pyridine (0.2 ml) in dry dichloromethane.
Yield: 0.250 g (40 %), m.p. 167 °C.
PMR ($CDCl_3$):
5.86 (d of d, $J_{trans}$ = 17 Hz, $J_{cis}$ = 10.8 Hz, 14-vinylic-CH), 5.56 (d, $J_{6,7}$ = 6.3 Hz, 6-CH), 5.48 (d, $J_{6,7}$ = 6.3 Hz, 7-CH), 5.32 (d of d, $J_{trans}$ = 17 Hz, $J_{gem}$ = 2 Hz, 15-vinylic-CH), 4.96 (d of d, $J_{cis}$ = 10.8 Hz, $J_{gem}$ = 2 Hz, 15-vinylic-CH), 4.28 (brt, 1-CH), 3.66 (t, $J_{CH-CH}$ = 6.3 Hz, $CH_2$-Cl), 3.28 (d, $J_{gem}$ ? 18 Hz, 12-α-CH), 2.44 (d, $CH_2$-CO), 2.36 (d, $J_{gem}$ = 18 Hz, 12-β-CH), 2.12 (m, $CH_2CH_2CH_2Cl$), 1.42, 1.36, 1.34, 1.32, 1.30, 1.14, 1.12 (s, 7X $CH_3$).

Example 10

7-β-(2-Oxopyrrolidin-1-yl)-1,9-isopropylidenyl(1,3-)dioxy-8,13-epoxy-labd-5,14-dien-11-one

The title compound is prepared by following the procedure of Example 8 using 7-β-(N-γ-chlorobutyryl)-amino-1,9-isopropylidenyl(1,3-)dioxy-8,13-epoxy-labd-5,14-dien-11-one (0.250 g) and sodium hydride (0.025 g, 55 % suspension) in dry dichloromethane. It is crystallised from pet.ether-dichloromethane.
Yield: 160 mg (69 %), m.p. 212 °C.
PMR ($CDCl_3$):
5.86 (d of d, $J_{trans}$ = 17 Hz, $J_{cis}$ = 10.8 Hz, 14-vinylic-CH), 5.58 (d, J = 4.2 Hz, 6-CH), 5.26 (d of d, $J_{trans}$ = 17 Hz, $J_{gem}$ = 2 Hz, 15-vinylic-CH), 4.98 (d of d, $J_{cis}$ = 10.8 Hz, $J_{gem}$ = 2 Hz, 15-vinylic-CH), 4.66 (d, J = 4.2 Hz, 7-CH), 4.1 (brt, 1-CH), 3.40-2.84 (m, N-$CH_2$), 3.12 (d, J = 18 Hz, 12-α-CH), 2.44 (d, J = 18 Hz, 12-β-CH), 2.16 (m, $COCH_2$), 1.48, 1.44, 1.36, 1.32, 1.28, 1.22, 1.20 (S, 7X $CH_3$).

Example 11

7-β-(2-Oxopyrrolidin-1-yl)-1α,9α-dihydroxy-8,13-epoxy-labd-5,14-dion-11-one

7-β-(2-Oxopyrrolidin-1-yl)-1,9-isopropylidenyl(1,3-)dioxy-8,13-epoxy-labd-5,14-dien-11-one (0.160 g) in 50 % acetic acid (12 ml) is stirred at room temperature for 24 hours. The reaction mixture is basified to pH 9 using 10 % sodium bicarbonate and extracted in dichloromethane. The organic layer is washed with water, brine and dried over anhydrous sodium sulphate and evaporated to dryness. The residue is chromatographed (2 % MeOH/$CHCl_3$) to obtain the desired compound. It is crystallised from pet.ether-dichloromethane.
Yield 100 mg (68.5 %), m.p. >290 °C.
PMR ($CDCl_3$):

5.84 (d of d, $J_{trans}$ = 17 Hz, $J_{cis}$ = 10.8 Hz, 14-vinylic-CH), 5.66 (d, J = 4.6 Hz, 6-CH), 5.40 (S, 1H, 9-OH), 5.28 (d of d, $J_{trans}$ = 17 Hz, $J_{gem}$ = 2 Hz, 15-vinylic-CH), 4.98 (d of d, $J_{cis}$ = 10.8 Hz, $J_{gem}$ = 2 Hz, 15-vinylic-CH), 4.90 (S, 1H, 1-OH), 4.50β (brt, 1-CH), 3.82 (d of d, N-CHα), 3.48 (d of d, N-CHβ), 2.40 (m, CO-CHα + 12-α-CH), 2.00 (c, CO-CHβ + 12-β-CH), 1.36, 1.30, 1.24, 1.20, 1.16 (S, 5X $CH_3$).

Example 12

7-β-(N-Chloroacetyl)amino-1,9-isopropylidenyl(1,3-)dioxy-8,13-epoxy-labd-5,14-dien-11-one

The title compound is prepared by following the procedure of Example 7 using 7-β-amino-1,9-isopropylidenyl(1,3-)dioxy-8,13-epoxy-labd-5,14-dien-11-one (1.6g), 2-chloroacetyl chloride (0.33 ml) and dry pyridine (0.66 ml) in dry dichloromethane (20 ml). It is crystallised from pet.ether-ethyl acetate.
Yield 1.0 g (52%), m.p. 210 °C.
PMR ($CDCl_3$):
6.55 (d, 1H, NH), 5.86 (d of d, $J_{trans}$ = 17 Hz, $J_{cis}$ = 10.8 Hz, 14-vinylic-CH), 5.38 (d of d, $J_{trans}$ = 17 Hz, $J_{gem}$ = 2 Hz, 15-vinylic-CH), 5.30 (brd, 6-CH + 7-CH), 4.90 (d of d, $J_{cis}$ = 10.8 Hz, $J_{gem}$ = 2 Hz, 15-vinylic-CH), 4.34 (brt, 1-CH), 4.15 (S, 1H, $CH_2Cl$), 3.26 (d, J = 18 Hz, 12-α-CH), 2.36 (d, J = 18 Hz, 12-β-CH), 1.45, 1.34, 1.30, 1.28, 1.26, 1.18, 1.12 (S, 7X $CH_3$).

Example 13

7-β-[N-Piperidinoacetyl]amino-1,9-isopropylidenyl(1,3-)dioxy-8,13-epoxy-labd-5,14-dien-11-one

A mixture of 7-β-[N-chloroacetyl]amino-1,9-isopropylidenyl(1,3-)dioxy-8,13-epoxy-labd-5,14-dien-11-one (0.5 g) and piperdine (0.11 ml) in dry chloroform is stirred overnight at 55 °C. The reaction mixture is concentrated under vacuum, residue diluted with water and extracted with chloroform.

The chloroform layer is washed with water, separated, dried over anhydrous sodium sulphate and evaporated to dryness. It is chromatographed using 2 % MeOH/$CHCl_3$ as the eluent to obtain the title compound as colourless thick oil.
Yield 0.400 g (72 %).
PMR ($CDCl_3$):
7.34 (brd, 1H, NH), 5.88 (d of d, $J_{trans}$ = 17 Hz, $J_{cis}$ = 10.8 Hz, 14-vinylic-CH), 5.34 (d of d, $J_{trans}$ = 17 Hz, $J_{gem}$ = 2 Hz, 15-vinylic-CH), 5.28 (brd, 2H, 6CH + 7-CH), 4.85 (d of d, $J_{cis}$ = 10.8 Hz, $J_{gem}$ = 2 Hz, 15-vinylic-CH), 4.32 (brt, 1-CH), 3.28 (d, J = 18 Hz, CO-CHα), 3.12 (d, J = 18Hz, 12-α-CH), 2.78 (d, J = 18 Hz, 12-β-CH), 2.55 (brs, 2H, N-$CH_2$), 2.48 (brs, 2H, N-$CH_2$), 2.42 (d, J = 18 Hz, CO-CHβ), 1.6 (brs, 6H, C-$CH_2$), 1.42, 1.36, 1.30, 1.26, 1.23, 1.15, 1.10 (S, 7X $CH_3$).

Example 14

7-β-[N-Dimethylaminoacetyl]amino-1,9-isopropylidenyl(1,3-)dioxy-8,13-epoxy-labd-5,14-dien-11-one

A mixture of 7-β-(N-chloroacetyl)amino-1,9-isopropylidenyl-(1,3-)dioxy-8,13-epoxy-labd-5,14-dien-11-one(0.400 g) and N,N-dimethylamine in alcohol (5 ml, 33 % in absolute ethanol) is stirred for four hours at room temperature.

The reaction mixture is evaporated to dryness. The residue obtained is diluted with water and then extracted in dichloromethane.

The organic layer is separated, washed with water, brine and dried over anhydrous sodium sulphate. It is evaporated to dryness to obtain the desired compound as colourless thick oil.
Yield 0.400 g (98 %).
PMR ($CDCl_3$):
7.20 (brd, 1H, NH), 5.34 (d of d, $J_{trans}$ = 17 Hz, $J_{cis}$ = 10.8 Hz, 14-vinylic-CH), 5.38 (d of d, $J_{trans}$ = 17 Hz, $J_{gem}$ = 2 Hz, 15-vinylic-CH), 5.30 (brd, 2H, 6-CH + 7-CH), 4.86 (d of d, $J_{cis}$ = 10.8 Hz, $J_{gem}$ = 2 Hz, 15-vinylic-CH), 4.30 (brt, 1-CH), 3.28 (d, J = 18 Hz, CO-CHα), 3.12 (d, J = 18 Hz, 12-α-CH), 2.80 (d, J = 18 Hz, 12-β-CH), 2.38 (d, J = 18 Hz, CO-CHβ), 2.32 (S, 6H, N-$CH_3$), 1.42, 1.38, 1.34, 1.28, 1.25, 1.18, 1.12 (S, 7X $CH_3$).

Example 15

7-β-[N-Piperidinoacetyl]amino-1α,9α-dihydroxy-8,13-epoxy-labd-5,14-dien-11-one

A mixture of 7-β-[N-piperidinoacetyl]amino-1,9-isopropylidenyl-(1,3)dioxy-8,13-epoxy-labd-5,14-dien-11-one (0.400 g), 2N hydrochloric acid (15 ml) and tetrahydrofuran (3 ml) is stirred for 4 hours. The reaction mixture is basified to pH 9 and extracted with dichloromethane. The dichloromethane layer is washed with water, brine and dried over anhydrous sodium sulphate and concentrated. The residue obtained is purified by column chromatography using 2 % MeOH/$CHCl_3$ as the eluent to obtain the desired product. It is crystallised from pet.ether-dichloro methane.
Yield 0.200 g (54,3 %), m.p. 185-186 °C
PMR ($CDCl_3$):
7.48 (d, 1H, NH), 5.94 (brS, 1H, 9-OH), 5.88 (d of d, $J_{trans}$ = 17 Hz, $J_{cis}$ = 10.8 Hz, 14-vinylic-CH), 5.44 (d of d, $J_{trans}$ = 17 Hz, $J_{gem}$ = 2 Hz, 15-vinylic-CH), 5.36 (d, J = 7 Hz 6-CH), 5.30 (d, J = 7 Hz, 7-CH), 5.08 (d of d, $J_{cis}$ = 10.8 Hz, $J_{gem}$ = 2 Hz, 15-vinylic-CH), 4.76 (brt, 1-CH), 3.88 (brS, 1H, 1-OH), 3.12 (d, J = 18 Hz, Co-CHα + 12-α-CH), 2.80 (d, J = 18 Hz, 12-β-CH), 2.66 (brd, J = 18 Hz, Co-CHβ + N-$CH_2$), 2.44 (brS, 4H, N-$CH_2$), 1.66 (brS, 6H, C-$CH_2$), 1.52, 1.30, 1.26, 1.24, 1.14 (S, 5X $CH_3$).

Example 16

7-β-[N-Dimethylaminoacetyl]amino-1α,9α-dihydroxy-8,13-epoxy-labd-5,14-dien-11-one

The title compound is prepared by following the procedure of Example 15 using 7-β-[N-α-(N,N-dimethylamino)-acetyl]amino-1,9-isopropylidenyl(1,3-)dioxy-8,13-epoxy-labd-5,14-den-11-one (0.400 h), 2N hydrochloric acid (10 ml) and tetrahydrofuran (4 ml). It is crystallised from pet.ether-dichloromethane.
Yield 0.250 g (68 %), m.p. 135-136 °C
PMR ($CDCl_3$):
7.38 (d, 1H, NH), 6.24 (brS, 1H, 9-OH), 5.92 (d of d, $J_{trans}$ = 17 Hz, $J_{cis}$ = 10.8 Hz, 14-vinylic-CH), 5.46 (d of d, $J_{trans}$ = 17 Hz, $J_{gem}$ = 2 Hz, 15-vinylic-CH), 5.36 (brd, 6-CH + 7CH), 5.08 (d of d, $J_{cis}$ = 10.8 Hz, $J_{gem}$ = 2 Hz, 5-vinylic-CH), 4.16 (brS, 1H, 1-OH), 3.18 (d, J = 18 Hz, CO-CHα), 3.12 (d, J = 18 Hz, 12-α-CH), 2.88 (d, J = 18 Hz, 12-β-CH), 2.60 (d, J = 18 Hz, CO-CHβ), 2.36 (brS, 6H, N-$CH_3$), 1.52, 1.36, 1.28, 1.24, 1.14 (S, 5X $CH_3$).

Example 17

7β-Acetylamino-8,13-epoxy-labd-5,14-dien-11-one

Following the procedure described in Examples 1 and 2, starting with 8,13 epoxy-7α-hydroxy-labd-5,14-dien-11-one and using aqueous solution of methylamine in place of hydrazine hydrate. 7-β-Amino-8,13-epoxy-labd-5,14-dien-11-one is prepared and treated with acetic anhydride and pyridine using methylene chloride as solvent, at room temperature for four hours. Work up of the reaction gives the solid residue which is purified by column chromatography over silica gel using eluent 10 % ethyl acetate in toluene to give the title compound, m.p. 96-97 °C.
PMR ($CDCl_3$):
7.76 (d, J = 9.97 Hz, NHCO), 5.99 (d of d, $J_{trans}$ = 17.09 Hz, $J_{cis}$ = 10.98 Hz, 14-vinylic CH), 5.34 (d of d, $J_{trans}$ = 17.09 Hz, $J_{gem}$ = 2 Hz, 15-vinylic-CH), 5.00 (d of d, $J_{cis}$ = 10.98 Hz, $J_{gem}$ = 2 Hz, 15-vinylic CH), 4.95 (S, 6-CH), 4.89 (d, J = 9.96 Hz, 7-CH), 2.74 (S, 9-CH), 2.71 (d, J = 15.87 Hz, 12β=H), 2.57 (d, J = 15.87 Hz, 12-α.H), 1.90 (S, $COCH_3$), 1.02, 1.08, 1.16, 1.25, 1.27 (S, 5X $CH_3$).

## Claims

**1.** A compound of the formula I

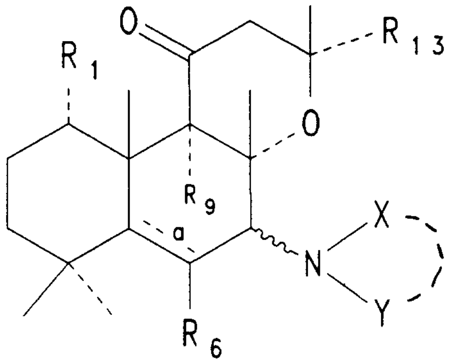

( I )

wherein

| | |
|---|---|
| a | denotes a single bond or a double bond between $C_5$ and $C_6$; |
| $R_1$ = | H or OH; |
| $R_9$ = | H or OH; or |
| $R_1$ and $R_9$ | taken together denote a group selected from |

O S

‖ ‖

-O-C-O, -O-C-O,

-O-C($C_1$-$C_4$-alkyl)$_2$-O- and

N($C_1$-$C_4$-alkyl)$_2$

|

-O-CH-O-;

$R_6$ =

O O O

‖ ‖ ‖

H, OH, -OC-($C_1$-$C_4$-alkyl), -OC-($C_1$-$C_4$-alkyl-$NH_2$), -O-C-($C_2$-$C_4$-alkenyl),

O

‖

-O-C-($C_2$-$C_4$-alkinyl)

or OCONH($CH_2$)$_m$-NH-O, wherein

| | |
|---|---|
| Q = | H, $C_1$-$C_4$-alkyl or aryl; |
| | m denotes an integer from 1 to 20; |
| $R_{13}$ = | vinyl, cyclopropyl or -$CHOHCH_2OH$; |

X = H or $(CH_2)_mH$,
Y = H, $(CH_2)_mH$,

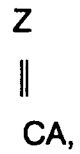

$(CH_2)_m$aryl or aryl, wherein
Z = O, S or NH and
A = H, $(CH_2)_mH$, $(CH_2)_mB$ or NHR' with
B = halogen, OH or

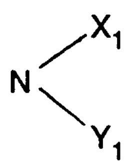 ;

and
R' = H, $C_1$-$C_4$-alkyl or aryl;
wherein
$X_1$ and $Y_1$ each represent an alkyl group or $X_1$ and $Y_1$ together with the nitrogen atom to which they are attached form a heterocycle which may contain an additional heteroatom N, O or S which is optionally substituted by 1, 2 or 3 identical or different alkyl or aryl radicals; or

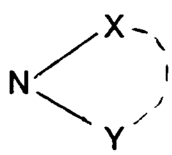

represents a group selected from

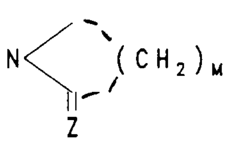 , 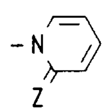 , 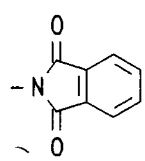

and

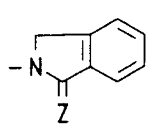

wherein M denotes an integer from 3 to 6 and Z denotes O or S;
and pharmaceutically acceptable salts thereof.

**2.** A compound as defined in claim 1 wherein $R_1$ and $R_9$ denote OH or $R_1$ and $R_9$ taken together denote a group selected from

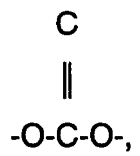
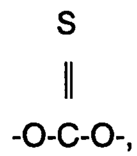

-O-($C_1$-$C_4$-alkyl)$_2$-O- and

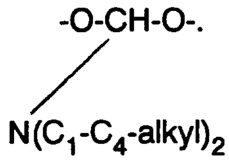

3. A compound as defined in claim 1 wherein $R_1$ and $R_9$ denote H.

4. A compound as defined in one or more of the claims 1 to 3, wherein $R_{13}$ denotes vinyl.

5. The compound as defined in one or more of the claims 1 to 4, which is 7-amino-8,13-epoxy-labd-5,14-dien-11-one.

6. The compound as defined in one or more of the claims 1 to 4, which is 7-acetylamino-8,13-epoxy-labd-5,14-dien-11-one.

7. A pharmaceutical composition which comprises a compound as defined in claim 1 as the active ingredient and a suitable carrier therefor.

8. Use of a compound as defined in claim 1 for the preparation of a medicament for treating cardiovascular diseases.

9. Use of a compound as defined in claim 1 for the preparation of a medicament for treating metabolic diseases.

10. A process for the preparation of a compound as defined in claim 1, which comprises
a) treating a compound of the formula II

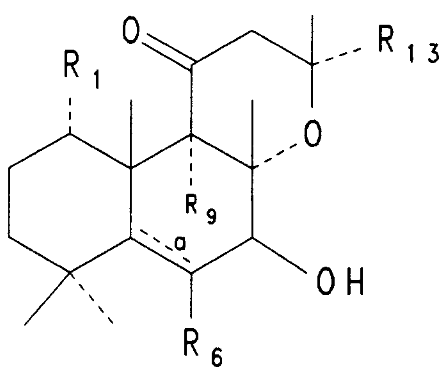

(II)

wherein
$R_6$ and $R_{13}$ have the previously defined meanings, except that $R_6$ = OH, and
$R_1$ and $R_9$ = H, or
when $R_9$ = H, $R_1$ = O($C_1$-$C_4$-alkyl),

O
||
OC-($C_1$-$C_4$-alkyl),

O-Si($C_1$-$C_4$)-alkyl)$_3$
or $R_1$ and $R_9$ taken together stand for -O-C($C_1$-$C_4$-alkyl)$_2$-O-,

-O-C-O, -O-C-O
|| ||
O S

or

N ($C_1$-$C_4$-alkyl)$_2$
|
-O-CH-O-

with a mixture of an imide, a tertiary phosphine and a dialkylazidodicarboxylate whereby the reactants in relation to the compound of the formula II are in a molar excess of 0.1 to 2.0, and thereafter treating the resulting compound of the formula IIIa

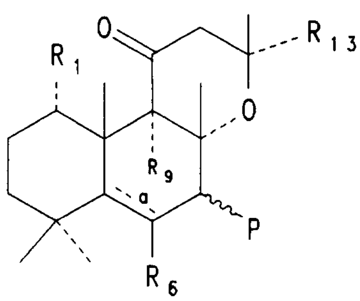

(III)

a) P= imido
b) P= azido

wherein P denotes imido and $R_1$, $R_9$, $R_6$ and $R_{13}$ have the same meaning as defined in formula II above, with an appropriate hydrolytic agent, or

b) treating a compound of the formula II above, with a mixture of an azide, a tertiary phosphine and a dialkylazidocarboxylate whereby the reactants in relation to the compound of the formula II are in a molar excess of 0.1 to 2.0, and thereafter treating the resulting compound of the formula IIIb above wherein P denotes azido and $R_1$, $R_9$, $R_6$ and $R_{13}$ have the same meaning as defined in formula II above, first with triphenyl phosphate and then with water or hydrogen in the presence of a catalyst or treatment with metal hydrides, and

c) optionally modifying the 7-$NH_2$-group of the 7-aminolabdane of the formula I having been obtained in steps a) or b), wherein $R_7$ denotes $NH_2$ and $R_1$, $R_9$, $R_6$ and $R_{13}$ have the same meaning as in formula I above, by conventional synthetic reactions.

European Patent Office

# EUROPEAN SEARCH REPORT

Application Number

EP 93 10 3605

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | JOURNAL OF MEDICINAL CHEMISTRY, vol. 31, no. 10, 1988, Washington, DC, US, pages 1872 - 1879 Y. KHANDELWAL, ET AL. 'Cardiovascular effects of new water-soluble derivatives of forskolin' * table I * ----- | 1,7,8 | C07D311/92 C07D405/04 C07D493/10 A61K31/35 |

TECHNICAL FIELDS SEARCHED (Int. Cl.5 )

C07D

The present search report has been drawn up for all claims

| Place of search | Date of completion of the search | Examiner |
|---|---|---|
| THE HAGUE | 18 MAY 1993 | RUSSELL F. ENGLISH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)